# EUROPÄISCHE PATENTSCHRIFT

(11) **EP 1 633 418 B1**
(45) Veröffentlichungstag und Bekanntmachung des Hinweises auf die Patenterteilung: **11.04.2012**
(21) Anmeldenummer: 04739731.0
(22) Anmeldetag: 09.06.2004
(51) Int. Cl.: A61M 5/24, A61M 5/20

(54) **ANTRIEBSVORRICHTUNG ZUM VORSCHUB EINES VORSCHUBELEMENTS**
DRIVE MECHANISM FOR ADVANCING AN ADVANCING ELEMENT
DISPOSITIF D'ENTRAINEMENT POUR AVANCER UN ELEMENT DE DEPLACEMENT EN AVANT

(30) Priorität: 13.06.2003 DE 10326766
(43) Veröffentlichungstag der Anmeldung: 15.03.2006
(73) Patentinhaber: Roche Diagnostics International AG, 6343 Rotkreuz (CH)
(72) Erfinder: HEINIGER, Hanspeter, CH-4932 Lotzwil (CH); JOST, Stefan, CH-3203 Mühleberg (CH); SIEGENTHALER, Roger, CH-3110 Münsingen (CH); WÜTHRICH, Heinz, CH-3048 Worblaufen (CH)
(74) Vertreter: Rentsch Partner AG
(86) Internationale Anmeldenummer: PCT/EP2004/006221
(87) Internationale Veröffentlichungsnummer: WO 2004/110532

(56) Entgegenhaltungen:
- EP-A- 0 897 728
- WO-A-94/13343
- WO-A-98/01171
- US-A- 5 244 465

## Beschreibung

Die vorliegende Erfindung betrifft eine Antriebsvorrichtung zum Vorschub eines Vorschubelements nach dem Oberbegriff des Anspruchs 1.

Bei Messverfahren z. B. zur Bestimmung von Blutzuckerwerten eines Diabetes-Patienten ist es üblich, eine Messsonde mit einer Dialysemembran in ein Gewebe des Patienten einzubringen, durch die eine Messflüssigkeit geführt wird, welche über die Membran mit dem Umgebungsmilieu des Gewebes in Austausch tritt. Die Messflüssigkeit kann z. B. aus einer Ampulle über ein Leitungssystem in die Sonde eingeleitet werden. Hierfür wird eine Antriebsvorrichtung zur Ausschüttung der Flüssigkeit aus der Ampulle an diese angeschlossen, die z. B. einen Kolben in der Ampulle auf einen Ampullenauslass zu antreibt. Es ist notwendig die Messflüssigkeit mit einem konstanten bestimmbaren Druck durch das Flüssigkeitssystem der Sonde zu leiten, um zuverlässige und reproduzierbare Messergebnisse zu erhalten. Die Antriebsvorrichtung ist zu diesem Zweck z. B. mit einem Federantrieb ausgestattet, beim dem eine Feder unter Vorspannung ein Vorschubelement gegenüber einem Gehäuse der Vorrichtung antreibt. Das Vorschubelement schiebt den Kolben in der Ampulle von seiner Ausgangsposition bis zu seiner Endposition am Auslass vor, wobei sich die Feder von einem komprimierten Anfangszustand in einen expandierten Endzustand entspannt und das Vorschubelement über die gesamte Vorschubstrecke von der Ausgangs- zur Endposition vorschiebt. Durch die Einstellung der Spannkraft der Feder, lässt sich der in der Ampulle erzeugte Druck bestimmen mit dem die Flüssigkeit in das Leitungssystem der Sonde eingeführt wird.

Bei solchen Antriebsvorrichtungen und Ausschüttverfahren ist es jedoch möglich, dass der erzeugt Druck mit fortschreitender Expansion der Feder nachlässt, da diese in einem stark komprimierten Zustand eine größere Druckkraft aufbringen kann, als in einem wenig komprimierten Zustand. Ferner müssen Federn verwendet werden, die einen Expansionsweg aufweisen, welcher der Gesamtvorschubstrecke des Vorschubelements entspricht, um die Flüssigkeit vollständig aus der Ampulle ausschütten zu können. Derartige Federn sind meist eher lang und bestimmen durch ihre Länge die Gesamtlänge der Antriebsvorrichtung.

Das aus der WO 94/13343 bekannte Injektionsgerät zeigt alle Merkmale des Oberbegriffs des Anspruch 1.

Es ist eine Aufgabe der Erfindung, eine Antriebsvorrichtung zum Vorschub eines Vorschubelements zu schaffen, das ein Vorschubelement mit einer einstellbaren und im Wesentlichen gleichbleibenden Kraft vorschiebt, eine kompakte Bauweise aufweist, mit einfachen Bauelementen auskommt und leicht zu bedienen ist.

Es ist eine weitere Aufgabe der Erfindung ein Verfahren zur Ausschüttung eines fluiden Produkts aus einem Behälter zu schaffen, bei dem das fluide Produkt mit gleichbleibendem Druck ausgeschüttet wird, der Druck während der Ausschüttung regulierbar ist und eine zuverlässige Ausschüttung sichergestellt ist.

Die Aufgaben der Erfindung werden durch eine Antriebsvorrichtung zum Vorschub eines Vorschubelements gemäß Anspruch 1 gelöst. Vorteilhafte Ausgestaltungen gehen aus den Unteransprüchen hervor.

Demnach weist eine Antriebsvorrichtung zum Vorschub eines Vorschubelements gegenüber einem Gehäuse der Vorrichtung eine Federeinrichtung zum Antrieb des Vorschubelements auf, durch die das Vorschubelement über eine Gesamtvorschubstrecke vorgeschoben werden kann, d. h. über die Strecke, über die das Vorschubelement in Bezug auf das Gehäuse maximal verschoben werden kann. Vorzugsweise wird als Federeinrichtung eine Spiralfeder verwendet. Die Federeinrichtung greift an einem Gegenelement und dem gegenüber dem Gegenelement beweglichen Vorschubelement innerhalb des Gehäuses an. Dabei muss die Federeinrichtung nicht notwendig direkt am Gegenelement oder am Vorschubelement angeordnet werden, es ist auch möglich, dass die Federeinrichtung am Gegen- und/oder Vorschubelement über ein Zwischenelement angreift, das die Kraft auf das jeweilige Element überträgt. Die Spiralfeder kann lediglich an den Elementen anliegen oder auch fest mit diesen verbunden sein.

In der Antriebsvorrichtung ist erfindungsgemäß eine Spanneinrichtung mit einem Spannelement zum Spannen der Federeinrichtung vorgesehen. Dabei ist ein vorbestimmter Abstand zwischen dem Gegenelement, welches durch das Spannelement gebildet wird und dem Vorschubelement einstellbar. Durch das Einstellen des vorbestimmten Abstands, d. h. das Spannen der Federeinrichtung, wird diese unter eine bestimmte Vorspannung gesetzt, wodurch sie eine bestimmte Spannkraft oder Druckkraft in Abhängigkeit des eingestellten Abstands auf das Vorschubelement bzw. das Gegenelement ausübt. Der vorbestimmte Abstand zwischen dem Gegenelement, welchen gleichzelg Spannelement ist, und dem Vorschubelement wird zum Aufbau einer Spannkraft vorzugsweise kleiner gewählt als die Länge der ungespannten Feder beträgt. Grundsätzlich ist es aber auch denkbar eine Spannkraft zu erzeugen, indem die Feder gedehnt wird, wobei ein vorbestimmter Abstand gewählt wird, der größer ist als die Länge der ungespannten Feder. Nach der Erfindung ist der Abstand zu Beginn des Vorschubs und nach einem Vorschub um eine Teilvorschubstrecke einstellbar, d. h. die Spanneinrichtung kann die Federeinrichtung während des Vorschubs des Vorschubelements nachspannen, ohne die Kraftübertragung und den Vorschub zu unterbrechen. Die Teilvorschubstrecke ist kürzer als die Gesamtvorschubstrecke des Vorschubelements. Vorzugsweise ist die Gesamtvorschubstrecke in mehrere, nicht notwendigerweise gleich lange Teilvorschubstrecken unterteilt, so dass eine Spannkraft während des Vorschubs des Vorschubelements über die Gesamtvorschubstrecke mehrere Male nachgespannt und kontrolliert werden kann. Dabei ist es auch möglich, die Kraft während eines Vorschubs oder nach einer Teilvorschubstrecke zu ändern und an neue Anforderungen anzupassen, indem ein anderer vorbestimmter Abstand eingestellt wird.

Bei dem beschriebenen Verfahren zur Ausschüttung eines fluiden Produkts aus einem Behälter durch einen Auslass in dem Behälter wird ein dem Auslass gegenüberliegender Kolben innerhalb des Behälters durch eine Antriebsvorrichtung mit einem Vorschubelement und einer Federeinrichtung in Richtung auf den Auslass .zu vorgeschoben. Erfindungsgemäß wird nach dem Vorschub des Kolbens um eine Teilvorschubstrecke, die kürzer ist als eine Gesamtvorschubstrecke des Kolbens, die Federeinrichtung durch eine Spanneinrichtung gespannt wird. Vorzugsweise wird zum Antrieb des Kolbens eine Antriebsvorrichtung verwendet wie sie oben beschrieben wurde, so dass der Kolben durch das Vorschubelement der Antriebsvorrichtung in Richtung auf den Auslass zu verschoben werden kann.

Dabei wird die Ampulle mit einem Gehäuse der Antriebsvorrichtung und der Kolben mit dem Vorschubelement verbunden, so dass die Spannkraft, bzw. Druckkraft der Federeinrichtung auf den Kolben und damit auf das fluide Produkt in der Ampulle übertragen und ein vorbestimmter Druck in der Ampulle erzeugt wird. Nachdem das Vorschubelement um eine Teilvorschubstrecke vorgeschoben wurde, wird die Federeinrichtung erneut gespannt, d. h. nachgespannt, indem wieder der vorbestimmte Abstand zwischen dem Gegenelement und dem Vorschubelement oder dem Spannelement eingestellt wird. Dadurch kann einem Druckabfall durch ein Nachlassen der Spannkraft aufgrund einer geringeren Federkompression vorgebeugt werden.

Durch die Erfindung kann beim Vorschub des Kolbens in der Ampulle über die gesamte Strecke, die der Kolben bis zum Ampullenauslass zurücklegt und die bevorzugt der Gesamtvorschubstrecke des Vorschubelements entspricht, ein konstanter Druck in der Ampulle erzeugt werden, so dass z. B. in einer Blutzuckermesssonde eine zuverlässig konstante Strömung der Messflüssigkeit erzielt werden kann oder der Flüssigkeitsdruck einer veränderlichen Flussgeschwindigkeit angepasst werden kann. Die Erfindung ist überall vorteilhaft einsetzbar, wo eine konstante Kraft auf ein sich langsam bewegendes Element ausgeübt werden soll. Es können einfache, kostengünstige Bauteile verwendet werden und der Zusammenbau der Antriebsvorrichtung ist durch die mechanische Funktionsweise der Antriebsvorrichtung unkompliziert und wenig zeitaufwendig.

Bei einer bevorzugten Ausführungsform der Erfindung ist das Gehäuse der Antriebsvorrichtung hülsenförmig ausgebildet. Das Vorschubelement ist stabförmig oder hülsenförmig ausgestaltet und ragt an einem Ende des Gehäuses aus dem Gehäuse hervor. Das Gegenelement kann z. B. von einem nach innen in das Gehäuse ragenden Vorsprung des Gehäuses, durch ein weiteres gegenüber dem Gehäuse entgegen der Vorschubrichtung feststehendes Element oder auch durch das Spannelement gebildet werden. Das Spannelement ist vorzugsweise ebenfalls hülsenförmig und weist eine senkrecht zur Vorschubrichtung verlaufende Angriffsfläche für die Federeinrichtung, d. h. die Spiralfeder auf. Die Spiralfeder kann z. B. zwischen der Angriffsfläche des Spannelement und einer senkrecht zur Vorschubrichtung verlaufende Angriffsfläche des Vorschubelements eingesetzt sein. Oder die Spiralfeder greift an einem gehäusefesten Vorsprung und der Angriffsfläche des Spannelements an, welches die Spannkraft auf das Vorschubelement überträgt.

Das Spannelement zum Spannen ist gegenüber dem Gehäuse und dem Vorschubelement in Richtung des Gegenelements beweglich, um den vorbestimmten Abstand einstellen zu können. Besonders bevorzugt ist das Spannelement beim Spannen gegenüber dem Vorschubelement in nur einer Richtung parallel zur Vorschubrichtung beweglich und ist in einer Gegenrichtung gegenüber dem Vorschubelement fest. So kann das Spannelement zur Einstellung des vorbestimmten Abstands gegenüber dem Vorschubelement bewegt werden und kann gleichzeitig die Spannkraft auf das Vorschubelement übertragen.

Zur Einstellung des vorbestimmten Abstands weist die Spanneinrichtung eine Dreheinrichtung mit wenigstens einem gegenüber dem Gehäuse drehbaren Drehelement auf. Das Drehelement ist z. B. hülsenförmig und ragt an einem Ende aus dem Gehäuses heraus, wobei aus dem anderen gegenüberliegenden Ende des Gehäuses das Vorschubelement vorgeschoben wird. Das Spannelement weist ein Gewinde und das das Drehelement ein Gegengewinde auf, das mit dem Gewinde des Spannelements zusammenwirkt, so dass durch eine Drehung des Drehelements das Spannelement gegenüber dem Vorschubelement bewegt und der vorbestimmte Abstand eingestellt werden kann.

In einem Beispiel welches nicht erfindungsgemäß ist, ist das Drehelement parallel zur Vorschubrichtung gegenüber dem Gehäuse fest aber gegenüber dem Gehäuse drehbar und bildet das Gegenelement für die Spiralfeder, d. h. es weist eine Angriffsfläche für die Spiralfeder auf. Das Spannelement ist gegenüber dem Drehelement verdrehgesichert, so dass es dessen Drehbewegung folgt, kann sich aber parallel zur Vorschubrichtung gegenüber dem Drehelement bewegen. Das Spannelement weist ein Innengewinde auf mit dem es auf einem Außengewinde des Vorschubelements sitzt. Das Vorschubelement ist gegenüber dem Gehäuse drehfest, so dass das Spannelement bei einer Drehung des Drehelements gegenüber dem Vorschubelement gedreht wird und sich aufgrund der Gewinde parallel zur Vorschubrichtung bewegt und ein vorbestimmter Abstand zwischen dem Spannelement und dem Gegenelement, bzw. dem Drehelement eingestellt werden kann. Dabei kann es von den Gewinden und einer Führungseinrichtung z. B. am Drehelement oder am Gegenelemente in Bewegungsrichtung geführt werden. Die Führungseinrichtung kann auch die Verdrehsicherung bilden. Besonders bevorzugt ist eine Rasteinrichtung vorgesehen, wodurch das Drehelement in nur einer Richtung drehbar ist oder die Drehung in nur einer Richtung auf das Spannelement übertragen wird. Dadurch kann sichergestellt werden, dass sich das Spannelement beim Spannen in nur einer Richtung gegenüber dem Vorschubelement in Richtung auf das Gegenelement bewegt.

Die Spiralfeder ist zwischen dem Spannelement und dem Drehelement, das als Gegenelement wirkt, eingesetzt. In ungespanntem Zustand ist die Spiralfeder nicht oder nur wenig komprimiert und das Spannelement liegt an einem Ende des Gehäuses an. Durch Drehung des Drehelements wird das Spannelement gegenüber dem Gehäuse ins Innere des Gehäuses auf dem Vorschubelement bewegt und komprimiert die Spiralfeder, wenn eine Widerkraft, z. B. ein Kolben einer Ampulle, am Vorschubelement anliegt. Die bei der Kompression entstehende Spannkraft wird durch das Vorschubelement, welches in Vorschubrichtung fest mit dem Spannelement verbunden ist, auf den Kolben übertragen. Der Kolben wird in der Ampulle auf den Auslass zu vorgeschoben, wobei sich das Spannelement zurück in Richtung des Endes des Gehäuses bewegt und sich der Abstand zwischen Spannelement und Gegenelement vergrößert.

Nachdem das Vorschubelement eine Teilvorschubstrecke überwunden hat, wird durch ein erneutes Drehen am Drehelement das Spannelement wieder in Richtung des Gegenelement, d. h. des Drehelements; bewegt und es wird der vorbestimmte Abstand oder ein neuer ausgewählter Abstand für einen anderen Druck in der Ampulle eingestellt. Beim Nachspannen für den gleichen vorbestimmten Abstand zwischen Spannelement und Gegenelement wird das Spannelement dann um die Länge der Teilvorschubstrecke auf dem Vorschubelement in Richtung des Gegenelements, bzw. Drehelements, bewegt. Es ist beim Nachspannen nicht erforderlich den Kraftübertrag zwischen Spiralfeder und Vorschubelement und damit zum Kolben zu unterbrechen, wodurch der Druck auf das fluide Produkt in der Ampulle kontinuierlich aufrecht erhalten werden kann. Das Nachspannen kann so oft wiederholt werden, bis der Kolben am Auslass der Ampulle angekommen ist und das Vorschubelement die Gesamtvorschubstrecke überwunden hat. Danach kann die Antriebsvorrichtung von der Ampulle abgenommen werden und für einen neuen Einsatz bei einer vollen Ampulle vorbereitet werden. Hierzu wird das Vorschubelement in eine Anfangsposition zurückgeführt, indem das Spannelement auf dem Vorschubelement zurück bewegt wird.

Bei einer Ausführungsform der Erfindung dogegen, ist das Spannelement gegenüber dem Gehäuse verdrehgesichert, aber in Längsrichtung des Gehäuses beweglich und bildet das Gegenelement der Antriebsvorrichtung. Das Drehelement der Dreheinrichtung ist in Längsrichtung gegenüber dem Gehäuse fest, aber gegenüber diesem drehbar und weist eine durch das Gehäuse verlaufende stab- oder hülsenähnliche Verlängerung mit einem Außengewinde auf. Das Spannelement, bzw. Gegenelement, weist ein Innengewinde mit dem es in das Außengewinde der Verlängerung des Drehelements eingreift, auf. Das Vorschubelement ist hülsenförmig ausgebildet, so dass es die Verlängerung des Drehelements, das Spannelement und die Spiralfeder in seinem Inneren aufnehmen kann. Die Spiralfeder ist zwischen einer senkrecht zur Vorschubrichtung verlaufenden Angriffsfläche des Vorschubelements und dem Spannelement als Gegenelement eingesetzt.

Durch Drehung des Drehelements gegenüber dem Gehäuse wird das Spannelement auf der Verlängerung des Drehelements in Richtung auf das Vorschubelement, d. h. die Angriffsfläche des Vorschubelements für die Spiralfeder, verschoben, wodurch der vorbestimmte Abstand zwischen dem Spannelement, bzw. dem Gegenelement, und dem Vorschubelement eingestellt werden kann. Dabei wird es durch die Gewinde und z. B. durch Führungseinrichtungen an einer Innenfläche des Gehäuses in Bewegungsrichtung geführt. Liegt eine Gegenkraft, wie etwa ein Kolben einer Ampulle, an dem Vorschubelement an, wird in der Spiralfeder eine Spannkraft erzeugt. Die Spannkraft wird auf den Kolben in der Ampulle übertragen, wobei sich das Vorschubelement in Vorschubrichtung aus dem Gehäuse heraus bewegt und das fluide Produkt durch den Auslass verdrängt wird. Dabei vergrößert sich der Abstand zwischen Vorschubelement und Spannelement und nachdem das Vorschubelement um eine Teilvorschubstrecke vorgeschoben wurde, wird durch erneutes Drehen des Drehelements das Spannelement wieder auf der Verlängerung des Drehelements auf die Angriffsfläche des Vorschubelements zu bewegt und der vorbestimmte Abstand eingestellt. Das Nachspannen kann wiederholt werden bis das Spannelement von einem Ende des Gehäuses, bzw. der Verlängerung des Drehelements, bis zum anderen Ende bewegt wurde und das Vorschubelement die Gesamtvorschubstrecke durchlaufen ist. Für einen erneuten Einsatz der Antriebsvorrichtung kann das Spannelement auf der Verlängerung wieder zurück bewegt werden.

Besonders bevorzugt ist bei dieser Ausführungsform der Erfindung eine Arretiereinrichtung vorgesehen, durch die das Vorschubelement gegenüber dem Gehäuse beim Spannen der Spanneinrichtung festgestellt werden kann. Durch das Feststellen des Vorschubelement wirkt beim Spannen der Feder eine Kraft entgegen und es kann eine Spannkraft aufgebaut werden, ohne dass eine Ampulle bereits an dem Gehäuse und das Vorschubelement am Kolben angebracht ist. In arretiertem Zustand des Vorschubelements wird das Spannelement durch Drehen des Drehelement auf das Vorschubelement zu bewegt und der vorbestimmte Abstand eingestellt und die Spannkraft erzeugt. Zur Übertragung der Spannkraft auf einen an das Vorschubelement angesetzten Kolben wird die Arretiereinrichtung gelöst und es kann ein Druck in der Ampulle aufgebaut werden.

Innerhalb dem variierenden Abstand zwischen dem Spannelement und dem Gegenelement oder dem Vorschubelement und dem Gegenelement, der durch den Vorschub des Vorschubelements um eine Teilvorschubstrecke entsteht, d. h. der variierenden Länge der komprimierten Spiralfeder, kann die Feder einen gleichbleibenden Druck ausüben. Die Längenänderungen der Feder in diesen Größenordnungen rufen keine merklichen Druckschwankungen hervor.

Durch besonders große Haftreibung zwischen dem Kolben und einer Ampullenwand, z. B. nach einer längeren Lagerung der Ampulle, ist es möglich, dass die Spannkraft der Spiralfeder, die für einen gewünschten Druck in der Ampulle notwendig ist, nicht ausreicht, um diese erhöhte Anfangsreibung zu überwinden. Es ist deshalb vorteilhaft bei der Antriebsvorrichtung eine Anstoßeinrichtung vorzusehen, mit der der Kolben zu Beginn des Vorschubs angestoßen werden kann. Die Anstoßeinrichtung kann eine Betätigungseinrichtung, wie etwas einen Druckknopf, und ein Stoßelement aufweisen, wobei durch Betätigung der Betätigungseinrichtung, d. h. durch Drücken des Druckknopfes, das Stoßelement in Vorschubrichtung aus dem Gehäuse gestoßen wird. Dabei kann in einer Ausführungsform der Druckknopf aus einem Ende des Gehäuses hervorragen und einen Fortsatz aufweisen, der durch das Gehäuse aus dem gegenüberliegenden Ende des Gehäuses herausragt. Bei einer anderen Ausführungsform kann das Stoßelement durch das Vorschubelement gebildet werden, das mit dem Druckknopf verbunden ist. Durch das Drücken des Knopfes soll keine starke Bewegung des Kolbens hervorgerufen werden, ein leichtes Ankicken des Kolbens genügt, um die erhöhte Anfangshaftreibung zu überwinden. Zwischen dem Druckknopf und dem Gehäuse kann eine Feder vorgesehen sein, um das Stoßelement nach seinem Anstoß wieder in seine Ausgangstellung zurückzubringen.

Mit der vorliegenden Erfindung kann in einfacher mechanischer Weise ein konstanter Druck in der Ampulle erzeugt werden. Im Vergleich zu herkömmlichen Antriebsvorrichtung ist es möglich, kürzere Spiralfedern zu verwenden und somit die Gesamtlänge der Antriebsvorrichtung zu vermindern. Die Antriebsvorrichtung kann auch mit einer Anzeige versehen sein, welche die Anzahl der Drehungen der Dreheinrichtung misst, um das Einstellen eines bestimmten Abstands zu erleichtern.

Die Erfindung wird an beispielhaften Ausgestaltungen an Hand der Zeichnungen 6 - 10 erläutert. In dieser stellen dar:
- Fig. 1:: eine perspektivische Explosionsansicht einer ersten Antriebsvorrichtung;
- Fig. 2:: einen Längsschnitt durch die erste Antriebsvorrichtung mit ungespannter Federeinrichtung;
- Fig. 3:: einen Längsschnitt durch die erste Antriebsvorrichtung mit einem eingestellten vorbestimmten Abstand zwischen Spannelement und Gegenelement;
- Fig. 4:: einen Längsschnitt durch die erste Antriebsvorrichtung mit vorgeschobenem Vorschubelement;
- Fig. 5:: einen Längsschnitt durch die erste Antriebsvorrichtung mit einer betätigten Stoßeinrichtung;
- Fig. 6:: eine perspektivische Explosionsansicht einer Ausführungsform einer Antriebsvorrichtung nach der vorliegenden Erfindung;
- Fig. 7a:: einen Längsschnitt durch die zweite Ausführungsform der Antriebsvorrichtung mit ungespannter Federeinrichtung;
- Fig. 7b:: eine Detailansicht einer Arretiereinrichtung einer Antriebsvorrichtung in arretiertem Zustand;

- Fig. 8a:: einen Längsschnitt durch die Ausführungsform der erfinderischen Antriebsvorrichtung mit einem eingestellten vorbestimmten Abstand zwischen Spannelement und Vorschubelement;
- Fig. 8b:: eine Detailansicht einer Arretiereinrichtung einer Antriebsvorrichtung in entriegeltem Zustand;
- Fig. 9:: einen Längsschnitt durch die Ausführungsform der erfinderischen Antriebsvorrichtung mit vorgeschobenem Vorschubelement und
- Fig. 10:: einen Längsschnitt durch die Ausführungsform der erfinderischen Antriebsvorrichtung mit einer betätigten Stoßeinrichtung.

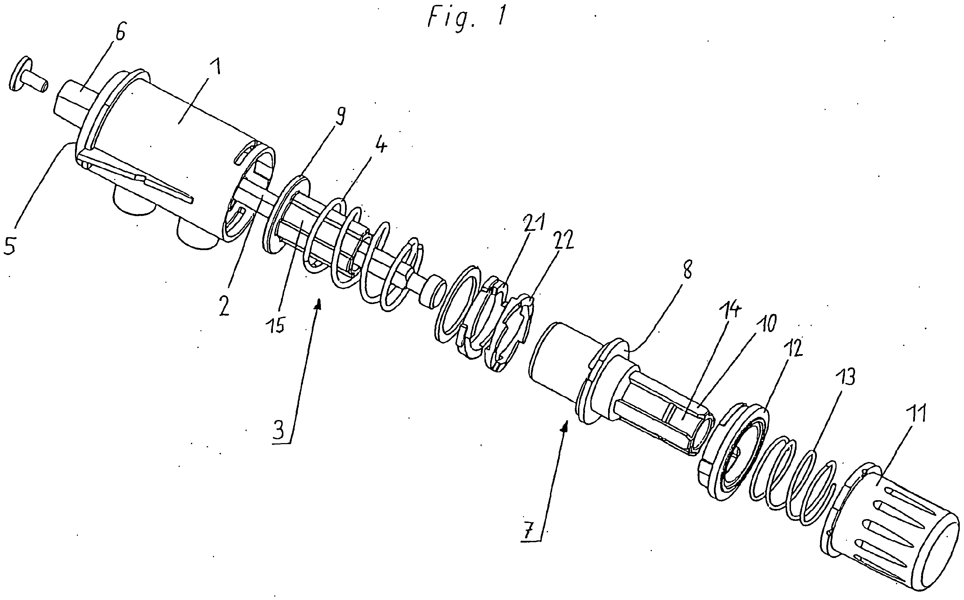

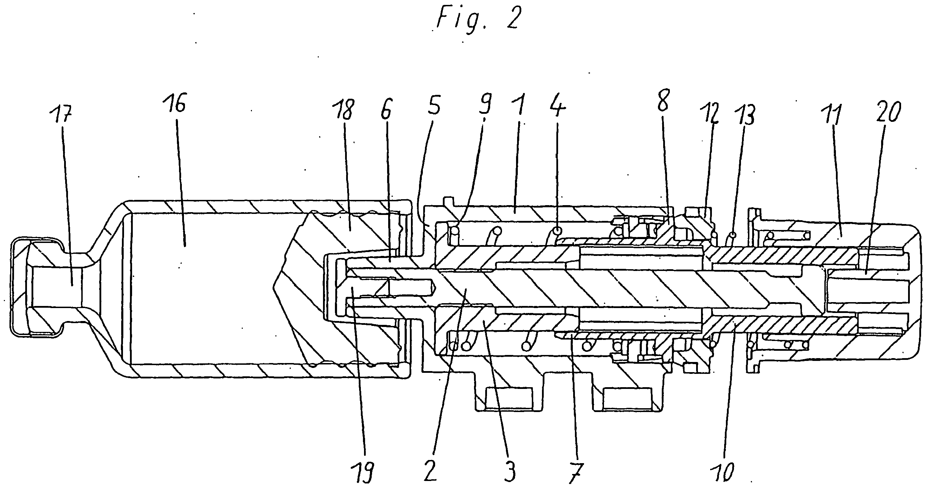

In Figur 1 ist eine Antriebsvorrichtung in einer Explosionsansicht und in Figur 2 in einem zusammengesetzten Zustand gezeigt. In einem hülsenförmigen Gehäuse 1 werden ein Vorschubelement in Form einer Gewindestange 2, ein hülsenförmiges Spannelement 3 und eine Federeinrichtung in Form einer Spiralfeder 4 eingesetzt. Das Gehäuse 1 weist an einem Ende eine das Gehäuse 1 eine abschließende Wand 5 mit einem Durchgang für das Vorschubelement 2 auf. Um den Durchgang ist ein rohrartiger Fortsatz 6 vorgesehen, auf den eine Ampulle 16 aufgesteckt werden kann (siehe Figur 2). In das gegenüberliegende Ende des Gehäuses 1 wird ein Drehelement 7 in das Gehäuse 1 eingesetzt, das eine senkrecht zur Umfangsrichtung des Gehäuse 1 verlaufende Schürze 8 aufweist. Die Schürze 8 bildet eine Angriffsfläche für die Spiralfeder 4. Das Drehelement 7 wird durch einen Haltering 12 am Gehäuse 1 gehalten. Das Spannelement 3 weist an seinem der Wand 5 des Gehäuses 1 zugewandten Ende eine senkrecht zur Umfangsfläche des Gehäuses 1 verlaufende Angriffsfläche 9 für die Spiralfeder 4 auf. Die Spiralfeder 4 ist zwischen der Angriffsfläche 9 und der Schürze 8 eingesetzt.

Das Drehelement 7 weist eine Verlängerung 10 auf, die aus dem Gehäuse 1 herausragt und auf der ein Druckknopf 11 aufgesetzt ist. Zwischen dem Haltering 12 und dem Druckknopf 11 ist eine Feder 13 eingesetzt. Die Verlängerung 10 weist auf ihrer Umfangsfläche in Längsrichtung verlaufende Führungsnuten 14 auf, in die Vorsprünge an einer Innenfläche des Druckknopfes 11 eingreifen. Der Druckknopf 11 ist daher gegenüber der Verlängerung 10 in Umfangsrichtung verdrehgesichert.

Weiter weist das Spannelement 3 auf seiner Außenmantelfläche in Längsrichtung verlaufende Führungsnuten 15 auf. In die Führungsnuten 15 greifen Vorsprünge an einer Innenmantelfläche des im Inneren des Gehäuses 1 liegenden Bereichs des Drehelements 7 ein. Das Spannelement 3 ist daher in Längsrichtung gegenüber dem Drehelement 7 in das Innere des Drehelements 7 hinein bewegbar. Gleichzeitig ist es gegenüber dem Drehelement 7 verdrehgesichert. Das Drehelement 7 wird gegenüber dem Gehäuse 1 in Längsrichtung fest angeordnet, kann aber durch Drehen des Druckknopfes gegenüber dem Gehäuse 1 gedreht werden. Die Gewindestange 2 wird an einem Ende des Gehäuses durch den rohrartigen Fortsatz 6 geführt und verläuft zentral in Längsrichtung des Gehäuses 1 durch das Gehäuse. Auf der Außenumfangsfläche der Gewindestange 2 ist ein Außengewinde vorgesehen. Auf der Innenmantelfläche des Spannelements 3 ist ein Innengewinde vorgesehen, das mit dem Außengewinde der Gewindestange 2 zusammenwirkt. Durch ein Verdrehen des Spannelements 3 mittels des Drehelements 7 gegenüber der Gewindestange 2 ist das Spannelement 3 daher auf der Gewindestange in Längsrichtung beweglich.

In Figur 2 ist die Antriebsvorrichtung in einer Ausgangsstellung gezeigt. Eine Ampulle 16 mit einem Auslass 17 für ein fluides Produkt wird mit ihrem, dem Auslass 17 gegenüberliegenden Ende an dem Fortsatz 6 des Gehäuses 1 der Antriebsvorrichtung angebracht. Der Fortsatz 6 ragt dabei teilweise in eine Aussparung eines Kolbens 18 hinein. Die Gewindestange 2 verläuft durch den Fortsatz 6 und weist an ihrem Ende in Richtung des Kolbens 18 einen Stempel 19 auf. In der Ausgangsstellung ist der Stempel 19 der Gewindestange 2 nicht in Kontakt mit dem Kolben 18 der Ampulle 16.

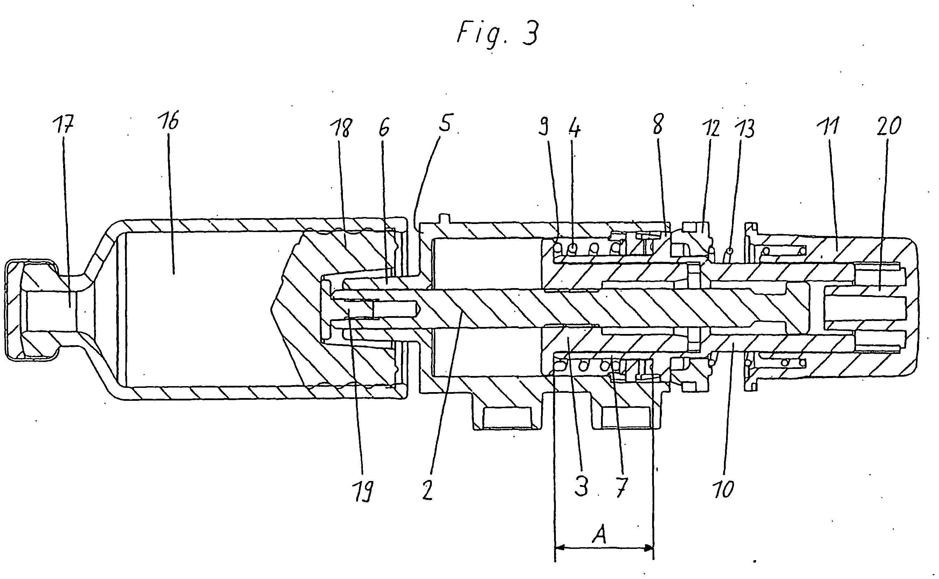

In Figur 3 wird ein vorbestimmter Abstand A zwischen der Angriffsfläche 9 des Spannelements 3 und der Schürze 8 des Drehelements 7 eingestellt. Hierfür wird der Druckknopf 11 gegenüber dem Gehäuse 1 in einer Richtung gedreht, wodurch auch das Drehelement 7 und somit auch das Spannelement 3 gegenüber dem Gehäuse 1 gedreht wird. Die Gewindestange 2 ist gegenüber dem Gehäuse 1 verdrehgesichert. Das Spannelement 3 bewegt sich daher bei einer Drehung des Druckknopfes 11 um die Gewindestange 2 und wird durch die zusammenwirkenden Gewinde in Richtung des Drehelements bewegt, das als Gegenelement für die Spiralfeder 4 wirkt. Dabei wird die Angriffsfläche 9 des Spannelements 3 von der Wand 5 des Gehäuses 1 entfernt und die Spiralfeder 4 auf eine Länge entsprechend dem Abstand A komprimiert und eine Spannkraft, bzw. Federkraft, aufgebaut. Die Federkraft wirkt gegen die gehäusefeste Schürze 8 und die Angriffsfläche 9 des Spannelements, welches die Kraft auf die Gewindestange 2 übertragt. Da das Drehelement 7 mit der Schürze 8 gegenüber dem Gehäuse feststeht wirkt die Federkraft in Vorschubrichtung der Gewindestange 2, bzw. des Kolbens 18 der Ampulle 16. Die Gewindestange 2 mit dem Stempel 19 wird zunächst soweit vorgeschoben, bis der Stempel 19 an einer senkrecht zur Umfangsfläche der Ampulle 16 verlaufenden Fläche des Kolbens 18 anliegt. Anschließend kann die Federkraft über den Stempel 19 auf den Kolben 18 übertragen werden, der dadurch innerhalb der Ampulle 16 vorgeschoben wird, da diese mit dem Gehäuse 1 der Antriebsvorrichtung fest ist.

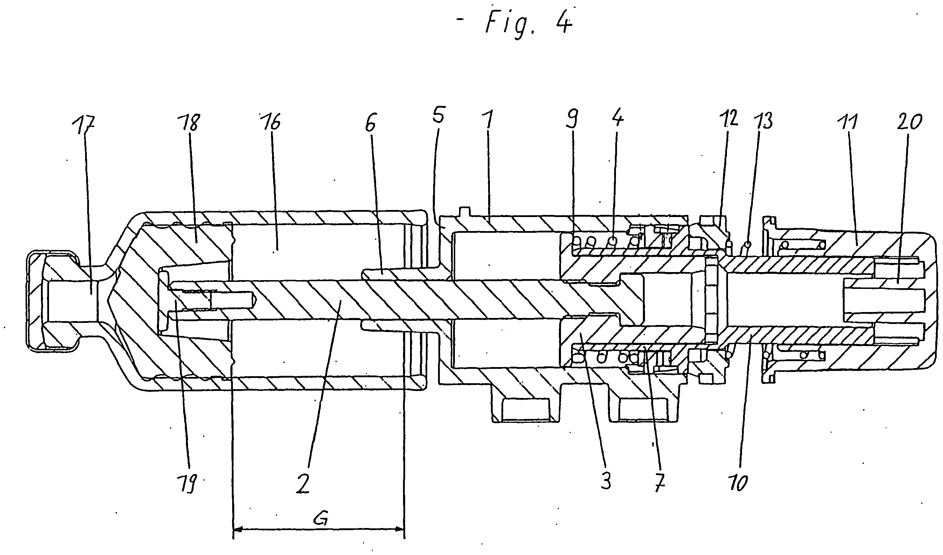

Durch die Federkraft wird die Gewindestange 2 mit dem Spannelement 3 innerhalb des Gehäuses 1 um eine Teilvorschubstrecke vorgeschoben und der Abstand A vergrößert sich, wodurch die Kompression der Feder abnimmt. Um die Feder erneut zu komprimieren und dadurch die Antriebsvorrichtung erneut zu spannen, kann durch Drehen des Druckknopfes 11 das Spannelement 3 erneut in Richtung auf das Drehelement 7 auf der Gewindestange 2 bewegt werden. Dabei wird die Kraftübertragung auf den Kolben 18 nicht unterbrochen. Dieser Vorgang kann nach mehreren Teilvorschubstrecken der Gewindestange 2 wiederholt werden, bis der Kolben 18 zu dem Auslass 17 der Ampulle 16 vorgeschoben ist, wie in Figur 4 gezeigt ist. In dieser Endstellung der Gewindestange 2 wurde die Gewindestange 2, bzw. der Kolben 18, um die Gesamtvorschubstrecke G vorgeschoben. Bei der Bewegung der Gewindestange 2 wird diese vorzugsweise durch Führungseinrichtungen an der Innenumfangsfläche des Fortsatzes 6 und durch das Spannelement geführt.

Im dargestellten Beispiel ist ferner eine Anstoßeinrichtung vorgesehen, die den Druckknopf 11, die Feder 13 und die Gewindestange 2 als Anstoßelement umfasst. Wie der Figur 2 zu entnehmen ist, ist die Feder 13 zwischen dem Haltering 12 und dem Druckknopf 11 gelagert, wobei ein Abstand zwischen dem Haltering 12 und dem ihm gegenüber liegenden Ende des Druckknopfes 11 vorgesehen ist. Der Druckknopf 11 weist einen zentral nach innen ragenden Steg 20 auf, der auf ein Ende der Gewindestange 2 gerichtet ist und senkrecht auf dieses Ende auftrifft, wenn die Antriebsvorrichtung in einer Anfangsstellung ist.

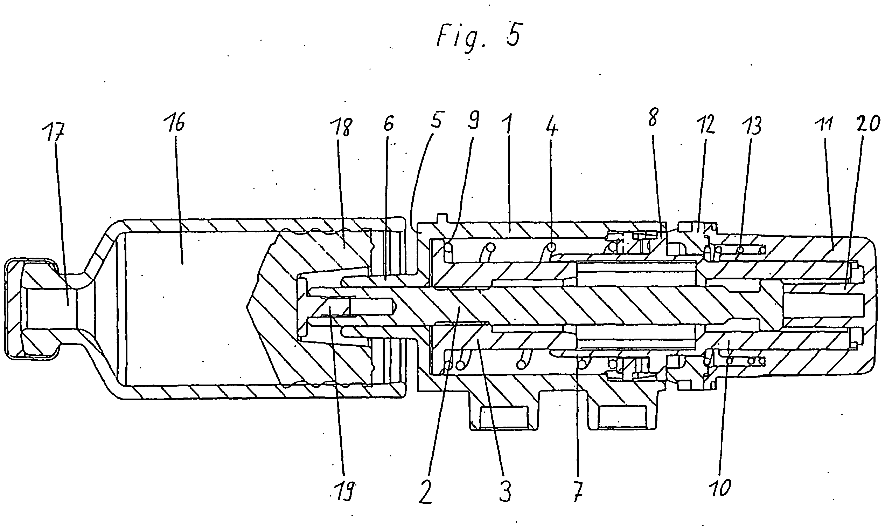

In Figur 5 ist der Druckknopf 11 in einer in Längsrichtung auf das Gehäuse 1 gedrückten Stellung gezeigt. Dabei wird die Gewindestange 2 von dem Steg 20 des Druckknopfes 11 in Vorschubrichtung vorgestoßen und überträgt den Stoß über den Stempel 19 auf den Kolben 18 der Ampulle 16. Durch diesen Stoß können Haftreibungen zwischen dem Kolben 18 und der Innenwand der Ampulle 16 überwunden werden. Der Druckknopf 11 wird durch die Federkraft der Feder 13 wieder entgegen der Vorschubrichtung von dem Gehäuse 1 in Längsrichtung fortbewegt. Nach dem Lösen des Kolbens 18 kann der Antrieb des Kolbens durch die Federkraft der Spiralfeder 4 der Antriebsvorrichtung erfolgen.

Weiter ist eine Rasteinrichtung vorgesehen, durch eine Klick-Rastung mit einem Rasten oder Klicken nach überschreiten eines diskreten Drehweges in eine der beiden Drehrichtungen möglich ist. Hierfür sind, wie in Figur 1 gezeigt, zur Übertragung der Drehbewegung des Drehelements 7 auf das Spannelement 3 Rastringe 21 und 22 vorgesehen. Die Rastringe 21 und 22 weisen zueinander komplementäre Stufen auf ihren senkrecht zur Umfangsfläche verlaufenden Flächen auf. An einem innen liegenden Rand der Schürze 8 ist ebenfalls ein in Längsrichtung gestufter Ring vorgesehen, der mit den Rastringen 21 und 22 zusammen wirkt. In einer Drehrichtung des Drehelements 7 stoßen die Stufen der Schürze 8 gegen Stufen des Rings 22, so dass der Ring 22 der Drehung folgt und mit seinen gegenüberliegenden Stufen über die Stufen des Rings 21 gezogen wird. Dabei entsteht ein Rasten bzw. Klicken. In der anderen Drehrichtung stoßen Stufen des Rings 22 gegen Stufen des Rings 21, so dass der Ring 22 der Drehbewegung nicht folgt und die Stufen der Schürze 8 über die Stufen des Rings 22 gezogen werden, wobei wieder ein Klicken entsteht. Eine solche Rasteinrichtung kann auch als eine Überlastsicherung bei Drehung ausgestaltet werden, wie in der Anmeldung DE 104 40 393 der Anmelderin beschrieben ist.

Bei der in den Figuren 1 bis 5 gezeigten Antriebsvorrichtung wird das Spannelement 3 zum Aufbau einer Federkraft in der Spiralfeder 4 durch die Dreheinrichtung entgegen der Vorschubbewegung in Richtung auf das Gegenelement, d.h., das Drehelement 7, bewegt.

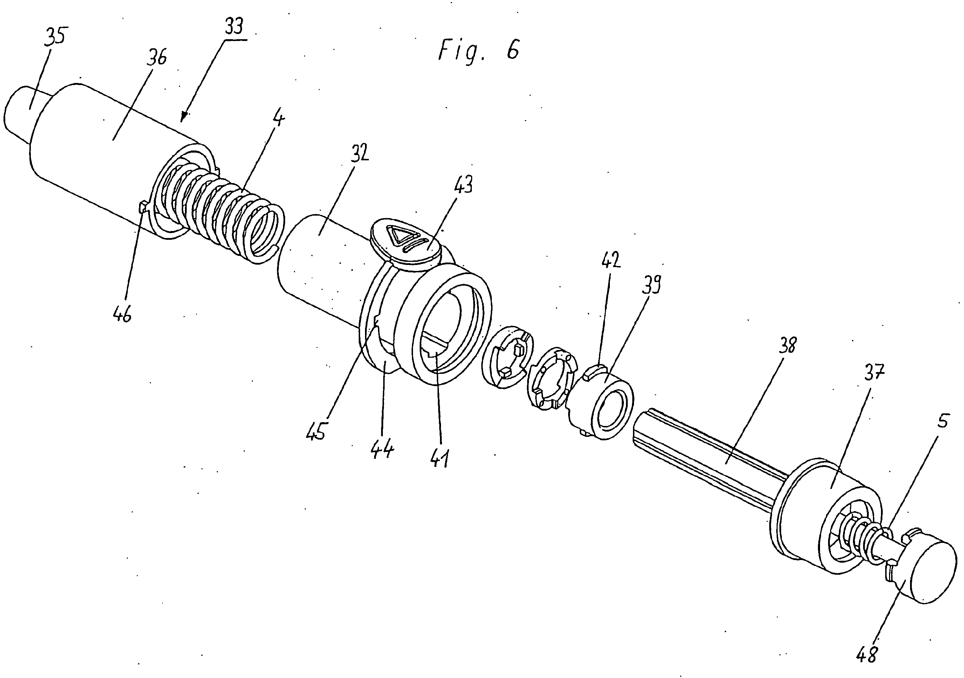

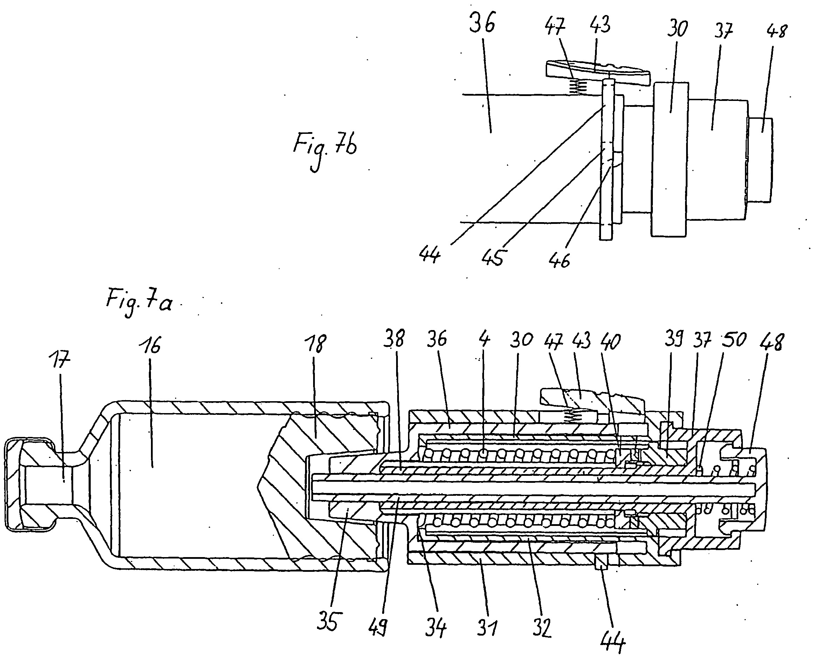

Bei der erfindungsgemäßen Ausführungsform, wie sie in den Figuren 6 bis 10 dargestellt ist, wird das Spannelement zum Aufbau der Federkraft von der Dreheinrichtung in Vorschubrichtung auf das Vorschubelement zu verschoben. In Figur 6 ist eine Explosionsansicht und in Figur 7a ein zusammengesetzter Aufbau der zweiten Ausführungsform der vorliegenden Erfindung gezeigt. Ein hülsenförmiges Gehäuse 30 ist doppelwandig ausgebildet, so dass es einen Zwischenraum zwischen einer äußeren Wand 31 und einer inneren Wand 32 bildet. Der Zwischenraum ist an einem Ende des Gehäuses 30, an welchem die Ampulle 16 angeschlossen wird, zugänglich und an dem gegenüberliegenden Ende abgeschlossen. Das Vorschubelement 33 dieser Ausführungsform ist im Wesentlichen hülsenförmig ausgebildet und verjüngt sich an einem Ende, so dass sich eine Angriffsfläche 34 und ein Fortsatz 35 mit verringertem Durchmesser bildet. Das hülsenförmige Vorschubelement 33 wird mit seiner Hülsenwand 36 in den Zwischenraum des Gehäuses 30 eingesetzt und ist innerhalb diesem entlang der Längsachse des hülsenförmigen Gehäuses 30 beweglich. Dabei kann eine Führungseinrichtung zur Führung der Längsbewegung vorgesehen werden.

Innerhalb des Gehäuses 30, d.h., innerhalb des durch die Innenwand 32 begrenzten Raumes, ist ein Drehelement 37 mit einer Verlängerung 38, die sich zentral durch das Gehäuse 30 erstreckt, eine Spiralfeder 4 und ein Spannelement 39, welches das Gegenelement für die Spiralfeder 4 bildet, vorgesehen. Das Drehelement 37 ragt an einem Ende des Gehäuses 30 heraus, das dem Ende, an dem das Vorschubelement 33 eingesetzt ist gegenüber liegt. Das Drehelement 37 ist durch komplementäre, ineinander greifende Stufen an dem Gehäuse 30 in Längsrichtung gesichert, so dass das Drehelement 37 in Längsrichtung gegenüber dem Gehäuse 30 nicht beweglich, jedoch gegenüber diesem verdrehbar ist. Die Verlängerung 38 erstreckt sich zentral durch das Innere des Gehäuses 30 und endet am gegenüberliegenden Ende des Gehäuses 30 vor dem Fortsatz 35 des Vorschubelements 33. Auf der Außenumfangsfläche der Verlängerung 38 ist ein Außengewinde vorgesehen. Das Spannelement 39 ist ebenfalls hülsenförmig ausgebildet und weist an seiner Innenumfangsfläche ein Innengewinde auf, das mit dem Außengewinde der Verlängerung 38 zusammenwirkt. Das Spannelement 39 weist eine senkrecht zur Vorschubrichtung verlaufende Fläche 40 auf und ist gegenüber dem Drehelement 37, d.h. auf der Verlängerung 38, in Längsrichtung des Gehäuses 30 verschiebbar. Es ist jedoch gegenüber dem Gehäuse 30 durch Führungsrillen 41 auf der Innenumfangsfläche der Innenwand 32 des Gehäuses 30 und Vorsprünge 42 auf der Außenumfangsfläche des Spannelements 39, welche in die Führungsrillen 41 eingreifen, gegenüber dem Gehäuse verdrehgesichert. Die Führungsrillen 31 verlaufen in Längsrichtung des Gehäuses 30 über die gesamte Länge der inneren Wand 32, so dass das Spannelement 39 über die gesamte Länge des Gehäuses in Längsrichtung verschiebbar ist.

Die Spiralfeder 4 ist zwischen die Angriffsfläche 34 des Vorschubelements 33 und die Fläche 40 des Spannelements 39 um die Verlängerung 38 angeordnet. An dem Ende des Gehäuses 30 aus dem das Vorschubelement 33 herausragt, wird eine Ampulle 16 an das Gehäuse 30 angesetzt. Dabei greift der Fortsatz 35 in eine Aussparung des Kolbens 18 der Ampulle 16 ein.

In Figur 7a ist die vorliegende Erfindung in einer Anfangsstellung gezeigt. Das Vorschubelement 33 wird von einer Arretiereinrichtung gegenüber dem Gehäuse 30 an einer Bewegung in Längsrichtung des Gehäuses gehindert. Die Arretiereinrichtung umfasst einen am Umfang des Gehäuses 30 angeordneten Auslöser 43 mit einem Ring 44, der das Vorschubelement 33 und die innere Wand 32 des Gehäuses 30 umfasst. Der Auslöser 43 ist senkrecht zur Längsrichtung des Gehäuses 30 beweglich. Der Ring 44 weist in seinem inneren Umfang Aussparungen 45 auf, die für Nasen 46 am Außenumfang des Vorschubelements 33 vorgesehen sind.

In arretiertem Zustand des Vorschubelements 33 wird der Auslöser 43 durch eine Feder 47 derart vorgespannt, dass die Nasen 46 des Vorschubelements 33 an einer Ringfläche neben den Aussparungen 45 zu liegen kommen und zwar auf einer dem Ampullenende des Gehäuses 30 abgewandten Seite, wie in Figur 7b dargestellt ist. Dadurch wird ein Vorschub des Vorschubelements 33 in Richtung der Ampulle durch den Anschlag der Nasen 46 an dem Ring 44 verhindert und das Vorschubelement 33 ist arretiert.

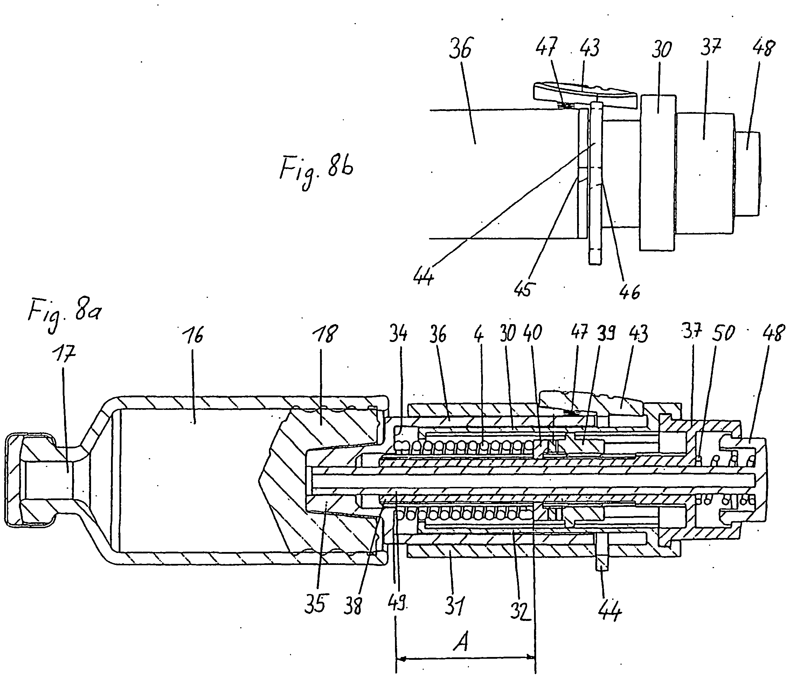

In Figur 8a wird das Spannen der Antriebsvorrichtung durch die Spanneinrichtung, d.h., das Spannelement, gezeigt. Durch Drehen des Drehelements 37 gegenüber dem Gehäuse 30 wird das Spannelement 39 durch das Zusammenwirken der Gewinde an dem Spannelement 39 und der Verlängerung 38 des Drehelement 37 in Richtung auf das Vorschubelement 33, bzw. auf dessen Angriffsfläche 34, zu verschoben. Dabei wird erfindungsgemäß das Spannelement 39 als Gegenelement für die Feder auf das Vorschubelement zu bewegt und ein vorbestimmter Abstand A zwischen dem Spannelement 39 und dem Vorschubelement 33, d.h., zwischen der Fläche 40 und der Angriffsfläche 34, eingestellt. Die Feder wird dadurch komprimiert und eine Federkraft erzeugt. Um die Federkraft auf den Kolben 18 der Ampulle 16 zu übertragen wird die Arretiereinrichtung durch Drücken des Auslösers 43 in Richtung der Umfangsfläche des Gehäuses 30 gelöst, so dass das Vorschubelement 33 freigegeben wird, indem sich der Ring 44 senkrecht zur Vorschubrichtung verschiebt, so dass die Aussparungen 45 gegenüber den Nasen 46 des Vorschubelements 33 zu liegen kommen. Durch die Federkraft der komprimierten Spiralfeder 4 wird das Vorschubelement 33 in Vorschubrichtung auf den Kolben zu bewegt, wobei sich die Nasen 46 durch die Aussparungen 45 bewegen, wie in Figur 8b gezeigt ist. Nach dem Auslösen des Vorschubelements 33 kann der Auslöser 43 durch die Federkraft der Feder 47 wieder in seine Ausgangsstellung zurückgebracht werden.

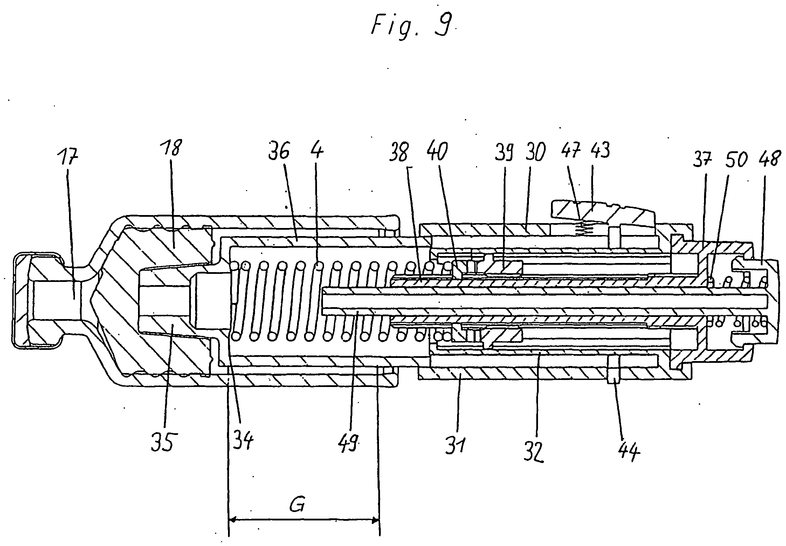

Das ausgelöste Vorschubelement 33 wird von der Federkraft der Spiralfeder 4 an den Kolben 18 der Ampulle 16 gepresst, so dass die Federkraft auf den Kolben 18 übertragen und dieser auf den Auslass 17 der Ampulle 16 hin vorgeschoben werden kann. Nach einer Teilvorschubstrecke des Vorschubelements 33 wird die Antriebsvorrichtung nachgespannt, indem das Drehelement 37 gedreht wird und das Spannelement auf der Verlängerung 38 weiter in Vorschubrichtung auf das Vorschubelement 33 zu bewegt wird. Dabei kann wieder der gleiche vorbestimmte Abstand A oder ein neuer Abstand eingestellt werden. Das Nachspannen der Antriebsvorrichtung kann mehrmals wiederholt werden, bis der Kolben 18 am Auslass 17 der Ampulle 16 anschlägt und das gesamte Fluid aus der Ampulle ausgegeben wurde, wie in Figur 9 gezeigt ist. Dabei wurde das Spannelement 39 über die Länge der Verlängerung 38 gegenüber dem Gehäuse vorgeschoben und das Vorschubelement hat die Gesamtvorschubstrecke G zurückgelegt.

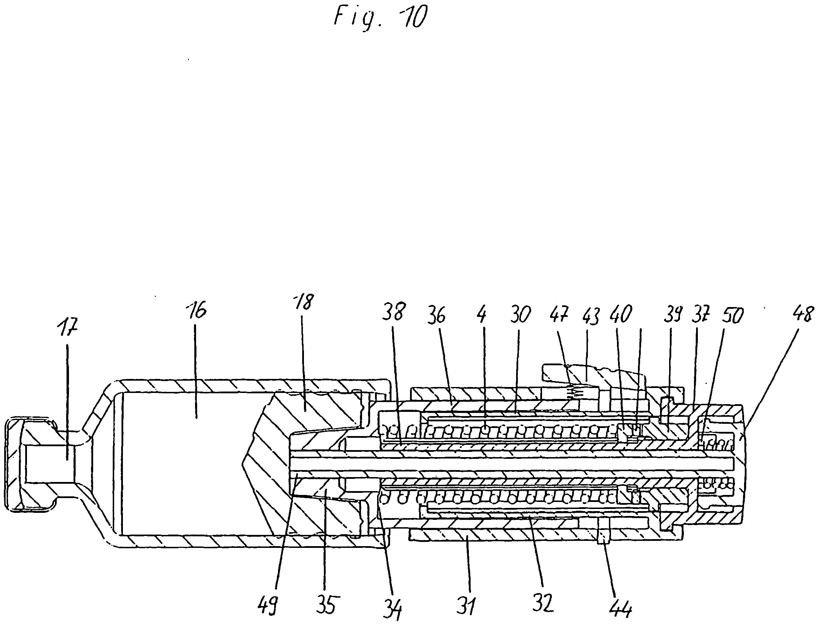

Auch kann eine Anstoßeinrichtung vorgesehen sein, wie in Figur 10 dargestellt ist. Die Anstoßeinrichtung weist eine Betätigungseinrichtung in Form eines Druckknopfes 48 auf, der zentral an einem Ende des Gehäuses 30 aus dem Gehäuse hervorsteht. In der gezeigten Ausführungsform ist der Druckknopf 48 an dem aus dem Gehäuse 30 hervorragenden Ende des Drehelements 37 angeordnet. Von dem Druckknopf 48 ragt zentral durch das Gehäuse 30 hindurch eine Durchführung 39 bis zum gegenüberliegenden Ende des Gehäuses ab. Die Durchführung 49 kann z.B. stangenförmig oder hülsenförmig sein und verläuft durch das Innere der Verlängerung 38 des Drehelements 37 und ragt in einer Anfangsstellung (vgl. Figur 7a) in Richtung auf den Kolben 18 über den Fortsatz 35 des Vorschubelements 33 hinaus. Eine Feder 50 hält den Druckknopf 48 in einer vorgespannten Position, in der die Durchführung 49 in einer zurückgezogenen Stellung ist.

Nach dem Spannen der Antriebsvorrichtung und dem Lösen der Arretiereinrichtung wird das Vorschubelement 33 mit der Federkraft der Spiralfeder 4 auf den Kolben 18 der Ampulle gestoßen, wie in Figur 8a gezeigt ist. Sollte die Federkraft nicht ausreichen, um eine anfänglich erhöhte Haftreibung des Kolbens 18 an der Innenwand der Ampulle 16 zu lösen, kann durch die Anstoßeinrichtung ein zusätzlicher Stoß auf den Kolben 18 ausgeübt werden. Hierfür wird der Druckknopf 48 gedrückt, wodurch die Durchführung 49 auf den Kolben 18 schlägt. Dadurch kann die erhöhte Haftreibung überwunden werden, so dass der Kolben allein durch die Federkraft der Spiralfeder 4 mittels des Vorschubelements 33 vorgeschoben werden kann.

In dem ersten Beispiel kann das Vorschubelement der Antriebsvorrichtung nach dem Vorschub um eine Teilvorschubstrecke gespannt werden, indem das Spannelement der Spanneinrichtung entgegen der Vorschubrichtung auf dem Vorschubelement in Richtung des Drehelements, bzw. des Gegenelements, verschoben wird. In der erfindungsgemäßen Ausführungsform jedoch erfolgt das Nachspannen der Antriebsvorrichtung durch eine Bewegung des Spannelements in Vorschubrichtung auf das sich ebenfalls in Vorschubrichtung bewegende Vorschubelement. Grundsätzlich sind noch weitere Ausführungsformen einer Antriebsvorrichtung der vorliegenden Erfindung denkbar, so lange ein vorbestimmter Abstand zwischen den Elementen, welche die Spiralfeder einfassen, durch eine Spanneinrichtung einstellbar ist nachdem ein Vorschubelement über eine Teilvorschubstrecke vorgeschoben wurde, die kleiner ist, als die Gesamtvorschubstrecke.

### Bezugszeichen

- 1: Gehäuse
- 2: Gewindestange
- 3: Spannelement
- 4: Spiralfeder
- 5: Wand
- 6: Fortsatz
- 7: Drehelement
- 8: Schürze
- 9: Angriffsfläche
- 10: Verlängerung
- 11: Druckknopf
- 12: Haltering
- 13: Feder
- 14: Führungsnut
- 15: Führungsnut
- 16: Ampulle
- 17: Auslass
- 18: Kolben
- 19: Stempel
- 20: Steg
- 21: Rastring
- 22: Rastring
- 30: Gehäuse
- 31: äußere Wand
- 32: innere Wand
- 33: Vorschubelement
- 34: Angriffsfläche
- 35: Fortsatz
- 36: Hülsenwand
- 37: Drehelement
- 38: Verlängerung
- 39: Spannelement
- 40: Fläche
- 41: Führungsrille
- 42: Vorsprung
- 43: Auslöser
- 44: Ring
- 45: Aussparung
- 46: Nase
- 47: Feder
- 48: Druckknopf
- 49: Durchführung
- 50: Feder
- A: Abstand
- G: Gesamtvorschubstrecke

## Patentansprüche

1. Antriebsvorrichtung zum Vorschub eines Vorschubelements (33) gegenüber einem Gehäuse (30) über eine Gesamtvorschubstrecke (G) zum Vorschub eines Kolbens in einer Ampulle zur Ausschüttung eines fluiden Produkts die eine Federeinrichtung (4) aufweist, die an einem Gegenelement (39) und dem gegenüber dem Gegenelement (39) beweglichen Vorschubelement (33) innerhalb des Gehäuses (30) angreift, wobei eine Spannvorrichtung mit einem Spannelement (39) zum Spannen der Federeinrichtung (4) vorgesehen ist, wobei ein vorbestimmter Abstand (A) zwischen dem Gegenelement (39) und dem Vorschubelement (33) nach Vorschub des Vorschubelements (33) um eine Teilvorschubstrecke, die kürzer ist als die Gesamtvorschubstrecke (G), einstellbar ist, **dadurch gekennzeichnet, dass** das Spannelement (39) gegenüber dem Gehäuse (30) verdrehgesichert ist und das Gegenelement bildet und durch eine Dreheinrichtung mit wenigstens einem gegenüber dem Gehäuse (30) drehbaren Drehelement (37) in Richtung des Vorschubelements (33) beweglich ist und das Spannelement (39) ein Gewinde und das Drehelement (37) ein Gegengewinde, das mit dem Gewinde des Spannelements (39) zusammenwirkt, aufweist.

2. Antriebsvorrichtung nach dem vorhergehenden Anspruch, **dadurch gekennzeichnet, dass** die Federeinrichtung durch eine Spiralfeder (4) gegeben ist, die zwischen dem Gegenelement (39) und dem Vorschubelement (33) eingespannt ist.

3. Antriebsvorrichtung nach einem der vorhergehenden Ansprüche, **dadurch gekennzeichnet, dass** der vorbestimmte Abstand (A) kleiner oder grösser ist als die Länge der ungespannten Federeinrichtung (4).

4. Antriebsvorrichtung nach einem der vorhergehenden Ansprüche, **dadurch gekennzeichnet, dass** eine Anstosseinrichtung mit einem Betätigungselement (48) und einem Stosselement (49) vorgesehen ist, wobei durch Betätigung des Betätigungselements (48) das Stosselement (49) in Vorschubrichtung stossbar ist.

5. Antriebsvorrichtung nach Anspruch 4, **dadurch gekennzeichnet, dass** das Betätigungselement (48) aus einem Ende des Gehäuses (30) hervorragt und einen Fortsatz (49) aufweist, der durch das Gehäuse (30) aus dem gegenüberliegenden Ende des Gehäuses (30) herausragt.

6. Antriebsvorrichtung nach einem der vorhergehenden Ansprüche, **dadurch gekennzeichnet, dass** eine Arretiereinrichtung (43, 44) zum lösbaren Feststellen des Vorschubelements (33) gegenüber dem Gehäuse (30) beim Spannen der Spanneinrichtung (4) vorgesehen ist.

## Claims

1. Drive device for advancing an advancing element (33) relative to a housing (30) over a total advancing distance (G) for advancing a piston in an ampoule for discharging a fluid product, comprising a spring device (4), that engages with a counter-element (39) and the advancing element (33) which is movable relative to the counter-element (39) inside the housing (30), wherein a tensioning device with tensioning element (39) for tensioning the spring device (4) is provided, wherein a predetermined distance (A) between the counter-element (39) and the advancing element (33) can be set after advancing of the advancing element (33) by a partial advancing distance that is shorter than the total advancing distance (G), **characterized in that** the tensioning element (39) is secured against rotation relative to the housing (30) and forms the counter-element and can be moved in the direction of the advancing element (33) by a rotary device with at least one rotary element (37) that is rotable with respect to the housing (30), and that the tensioning element (39) has a thread and that the rotary element (37) has a mating thread, that interacts with the thread of the tensioning element (39).

2. Drive element according to the preceding claim, **characterized in that** the spring device is given by a helical spring (4), which is tensioned between the counter-element (39) and the advancing element (33).

3. Drive element according to one of the preceding claims, **characterized in that** the predetermined distance (A) is smaller or greater than the length of the untensioned spring device (4).

4. Drive element according to one of the preceding claims, **characterized in that** a striker device with an actuating element (48) and a pusher element (49) is provided, wherein the pusher element (49) is able to be pushed in the advancing direction by actuating the actuating element (48).

5. Drive device according to claim 4, **characterized in that** the actuating element (48) protrudes from one end of the housing (30) and has an extension (49), which protrudes through the housing (30) from the opposite end of the housing (30).

6. Drive device according to one of the preceding claims, **characterized in that** a locking device (43, 44) is provided for releasably fixing the advancing element (33) relative to the housing (30) upon tensioning of the tensioning device (4).

## Revendications

1. Dispositif d'actionnement pour un mouvement d'avance d'un élément de mouvement d'avance (33) vis-à-vis d'une carcasse (30) à travers d'une distance de mouvement d'avance totale (G) pour le mouvement d'avance d'un piston à l'intérieur d'une ampoule pour la distribution d'un produit fluidique, ayant un dispositif de ressort (4) qui agit, à l'intérieur de la carcasse (30), sur un élément contrepartie (39) et sur l'élément de mouvement d'avance (33) qui est mobile en face de l'élément contrepartie (39), un dispositif de serrage avec un élément de serrage (39) pour serrer le dispositif de ressort (4) étant prévue, une distance (A) prédéterminée entre l'élément contrepartie (39) et l'élément de mouvement d'avance (33) pouvant être ajustée d'une distance partielle de mouvement d'avance qui est plus courte que la distance de mouvement d'avance totale (G), après le mouvement d'avance de l'élément de mouvement d'avance (33), **caractérisé en ce que** l'élément de ressort (39) est sécurisé contre une torsion par rapport à la carcasse (30) et forme l'élément contrepartie et est mobile dans la direction de l'élément de mouvement d'avance (33) à l'aide d'un dispositif de rotation avec au moins un élément de rotation (37) qui est rotatif par rapport à la carcasse (30) et l'élément de ressort (39) a un filetage et l'élément de rotation (37) a un filetage contrepartie qui agit conjointement avec le filetage de l'élément de ressort (39).

2. Dispositif d'actionnement selon la revendication précédente, **caractérisé en ce que** le dispositif de ressort est donnée par un ressort en spirale (4) enserrée entre l'élément contrepartie (39) et l'élément de mouvement d'avance (33).

3. Dispositif d'actionnement selon l'une des revendications précédentes, **caractérisé en ce que** la distance prédéterminée (A) est plus courte ou plus longue que la longueur du dispositif de ressort (4) en état relaxé.

4. Dispositif d'actionnement selon l'une des revendications précédentes, **caractérisé en ce qu'**un dispositif d'impulsion avec un élément d'actionnement (48) et un élément de poussée (49) sont prévus, l'élément de poussée (49) pouvant être poussé dans la direction de mouvement d'avance en actionnant l'élément d'actionnement (48).

5. Dispositif d'actionnement selon la revendication 4, **caractérisé en ce que** l'élément d'actionnement (48) fait saillie d'une extrémité de la carcasse (30) et comporte un prolongement (49) qui fait saillie de l'extrémité opposée de la carcasse (30).

6. Dispositif d'actionnement selon l'une des revendications précédentes, **caractérisé en ce qu'**un dispositif d'arrêt (43, 44) est prévu afin d'arrêter l'élément de mouvement d'avance (33) par rapport à la carcasse (30) pendant le serrage du dispositif de ressort (4).
